# EUROPEAN PATENT SPECIFICATION

(11) **EP 3 405 218 B1**
(45) Date of publication and mention of the grant of the patent: **06.09.2023**
(21) Application number: 17740913.3
(22) Date of filing: 20.01.2017
(51) Int. Cl.: C11D 3/48, C11D 1/62, C11D 3/34, C11D 3/386, A61L 2/18, A01N 33/12, A01N 63/50

(54) **SANITISING COMPOSITION**
DESINFEKTIONSZUSAMMENSETZUNG
COMPOSITION DÉSINFECTANTE

(30) Priority: 22.01.2016 AU 2016900195
(43) Date of publication of application: 28.11.2018
(73) Proprietor: Novapharm Research (Australia) Pty Ltd, Rosebery, NSW 2018 (AU)
(72) Inventor: SAVA, Alex, Paddington New South Wales 2021 (AU)
(74) Representative: Jacob, Reuben Ellis
(86) International application number: PCT/AU2017/050042
(87) International publication number: WO 2017/124150

(56) References cited:
- WO-A1-01/76647
- WO-A1-97/43384
- WO-A1-2006/005721
- FR-A1- 2 169 752
- US-A- 5 489 531
- US-A- 6 010 996
- US-A1- 2001 025 018
- US-A1- 2002 082 178
- US-A1- 2004 071 653

## Description

### FIELD OF THE INVENTION

This invention relates to methods suitable for High Level Disinfection ("HLD")¹ at close to ambient temperatures of medical instruments. Compositions for use in methods according to the invention are also effective cleaning agents for digesting biological contaminants. Most surprisingly compositions for use in methods according to the invention can clean and disinfect concurrently and it is believed will find most use for simultaneous cleaning and disinfection. The compositions are suitable for high level disinfection of flexible endoscopes and are herein described with particular reference to that use, but it will be understood that the compositions are equally suitable for treatment of a multitude of other instruments such as heat labile colonoscopes, laparascopes, ultrasound probes, other surgical, medical, biopsy, dental and such like instruments, parts of such instruments and similar paraphernalia (hereinafter collectively referred to as "instruments"). When used to clean and disinfect instruments, the instruments can be "reprocessed" (that is to say be cleaned, disinfected and readied suitable for re-use) more quickly and at lower temperatures than is possible with presently used processes, and with substantial energy savings. The methods are also applicable for treatment of instruments which are required merely to be sanitised for example hair-dressing tools, certain beauty parlour equipment, and the like (these examples are not according to the invention). It will be understood that although the invention is herein described with reference to its use for High Level Disinfection as per TGO54, it may be modified to provide lower levels of disinfection not according to the invention such as "Intermediate Level Disinfection", "Hospital Grade Disinfection", "Safe to handle' or as a Sanitiser if the intended use and applicable standards permit. Compositions for use in methods not according to the invention are also useful for cleaning and/or disinfection of other surfaces in hospitals, medical and dental practices, nursing homes or the like - for example chamber pots, trays, instrument transport trolleys and other large equipment - and for cleaning and/or disinfection pharmaceutical plants, food preparation areas, food utensils, dispensing equipment, cool rooms and the like, or fabrics and the like such as are treated in hospital laundries.
¹As defined in Therapeutic Goods Order No 54 of the Australian Therapeutic Goods Act 1989 (https://www.comlaw.gov.au/Details/F2009C00327)

### BACKGROUND OF THE INVENTION

### Prior Art

By way of example of prior art, endoscopes are increasingly being used in medical diagnosis and therapy. When used as directed endoscopes can become grossly soiled and massively contaminated with microorganisms which are present in non-sterile areas of the body, on the mucous membrane, and in the blood. Accordingly the instruments must be thoroughly cleaned and disinfected after each use. Endoscopes are precision instruments which are made from a combination of materials. They are difficult to clean in view of the sensitivity of the materials involved to chemical attack and because they have narrow lumens making access to and cleaning of interior surfaces difficult.

Unlike cheaper and smaller medical instruments that are reprocessed in central sterile supply departments with typical turnaround time of 24 hrs, flexible endoscopes are typically "reprocessed" in an Endoscopy Unit with a turnaround time of 30-60mins. Quick reprocessing is highly desired because of the relatively high capital cost of such instruments and the relatively short time required on average for their clinical use with each patient. Currently, reprocessing involves a sequential three step process. The first step, a "Cleaning Step", is usually conducted in two parts. In the first part "pre-cleaning", the endoscope after withdrawal by the clinician, undergoes a pre-clean at or near the bedside during which gross contamination is wiped from the instrument with a cloth soaked in enzymatic solution and then in a second part it is brushed/syringed/scrubbed clean with a cleaning solution typically comprising a suitable surfactant or enzyme / surfactant combination following a specified scrubbing protocol to ensure that all relevant external and internal surfaces are cleansed. When reprocessed in AERs the cleaning might be repeated with the same or different combination of surfactants and or enzymes. Ultimately, cleaning must be adequate to meet the standards set down by ISO 15883 (which is, or corresponding national standards of which are, internationally accepted as the standard to be obtained during reprocessing). The pre-cleaning and subsequent cleaning are herein considered collectively as The First Step of reprocessing.

The Cleaning step is followed by a second step, a "Rinsing Step" in which the instrument is thoroughly rinsed free of detergent, enzymes, and other residues which if not removed would be detrimental to the third ("Disinfection" ) step, and render it ineffective. For example, three thorough rinses are required to ensure that residuals on the pre-cleaned endoscope do not interfere with 400-600ppm of peracetic acid- one of the most popular endoscope disinfectants.

In a third step, the "Disinfection Step", the instrument is either sterilised in a steam autoclave (if not heat sensitive) or submerged in a bath with disinfectants able to achieve High Level Disinfection (e.g. peracetic acid, glutaraldehyde). The Cleaning, Rinsing and Sterilization steps may all be conducted sequentially in an Automatic Endoscope Reprocessor ("AER) before the instrument is dried and removed from the AER for reuse. Alternatively the pre-cleaned endoscope can be further manually cleaned in the same or a different bath of a cleaning solution, then removed from the bath to be manually thoroughly and repetitively rinsed manually in a second step, and finally is transferred to a disinfecting bath for manual high level disinfection in a third step. Whether the three consecutive steps are conducted in an AER or manually, at least three separate sequential processing steps are required before reuse of the endoscope.

A full understanding of the present invention requires insight into the difficulties of each of these three steps which are further described below.

### The cleaning step

As stated in "SCNA guidelines for use of High Level Disinfectants & Sterilants for Reprocessing Flexible Gastrointestinal Endoscopes": "Meticulous manual cleaning of all instruments must precede exposure to any high-level disinfectant or sterilant (Petersen et al., 2011*;* SGNA, 2012*). Inadequate cleaning of instruments has been reported as one factor responsible for transmission of infection by flexible endoscopes (ASGE Standards of Practice Committee et al., 2008; Rutala et al., 2008). This process significantly reduces the organic and microbial challenge to the high-level disinfectant or sterilant and is a vital step in preventing biofilm (*Alfa & Howie, 2009). A detailed cleaning protocol for endoscopes is found in SGNA's Standards of Infection Control and Reprocessing of Flexible Gastrointestinal Endoscopes (2012*)."²*
²SCNA guidelines for use of High Level Disinfectants & Sterilants for Reprocessing Flexible Gastrointestinal Endoscopes at page 9 see https://www.sgna.org/Portals/0/Issues/PDF/Infection-Prevention/6_HLDGuideline_2013.pdf

When manually cleaned in a bath, the brushing and syringing aerosolises the washing liquor and bacteria in the bath resulting in gross contamination of air and environmental surfaces of the endoscopy units. Such contaminated air is believed to be the most probable sources of re-infecting reprocessed instruments stored in the room causing incidents similar to that reported in the "UCLA incident" ³.
³see http://jama.jamanetwork.com/article.aspx?articleid=1911326

It is noteworthy that the pre-soak is not passive. Staff are instructed to syringe detergent liquor through all the lumens, to brush biopsy channels, valves etc. A colonoscope, for example, requires up to 14 manual brushing-syringing-plugging-unplugging operations, for cleaning. PPE recommended for use during pre-cleaning and cleaning includes gowns, gloves, protective eyewear, and or face protection.⁴
⁴SCNA guidelines for use of High Level Disinfectants & Sterilants for Reprocessing Flexible Gastrointestinal Endoscopes. Page 8. Similar standards are applicable internationally.

Enzyme containing detergents are significantly more efficient than detergents alone in removing stubborn water insoluble and proteinaceous soils and are currently the industry standard. Products such as 3M's RMEC^{®}, Steris's Prolystica^{®}, J&J's Cidezyme^{®} which involve a combination of enzymes and surfactants satisfactorily clean surfaces and meet the requirements ISO 15883. However they do not solve the problems addressed by our prior patent application Patent application WO 01/76647 A1 discussed hereinafter which further included a quaternary biocide in the cleaner. Whilst a number of quaternary biocide (hereinafter abbreviated to "quat") containing detergents, with or without enzymes, are marketed for cleaning and disinfection of medical devices, none of these quat containing products have the ability to clean to the level anticipated by ISO15883 (that involves cleaning a simulated soils indicator complying with ISO15883 e.g. Browne STF Load Check strip). Cleaning after pre-cleaning typically requires 5-7 mins in an AER and 10-15 mins when done manually. No product complying with the requirements for cleaning efficacy of ISO15833 offers or provides High Level Disinfection.

### The rinsing Step

Since components of the cleaning detergents interfere with the sterilisation and disinfection actives, a thorough rinsing is required between cleaning and disinfection steps. In the second step the instrument is thoroughly rinsed free of detergent, enzyme, and other residues. Instruments reprocessed in Automatic Endoscope Reprocessors ("AER's) typically undergo at least 3 rinse cycles in this step. Instruments not reprocessed in an AER are typically rinsed multiple. Rinsing typically takes up to 10-15 minutes in manual reprocessing and 6-10 minutes in AERs

### The Disinfection step

In the last decade, there has arisen a particular concern to avoid transmission of very serious and sometimes fatal diseases such as may be carried in blood and tissue, for example hepatitis B, HIV, and other infections and for heat sensitive instruments, High Level Disinfection is the minimum requirement to ensure avoidance of such transmissions. As set out for example in *"Guideline for Use of High Level Disinfectants & Sterilants for Reprocessing Flexible Gastrointestinal Endoscopes"* by the Society of Gastroenterology Nurses and Associates Inc⁵ ,
⁵https://www.sgna.org/Portals/0/Issues/PDF/Infection-Prevention/6_HLDGuideline_2013.pdf the disinfection step typically involves use of high level disinfectants the most commonly used being Peracetic Acid ("PAA"), Glutaraldehyde, Orthophalaldehyde ("OPA"), or concentrated Hydrogen Peroxide.

Up to now *"All high-level disinfectants or sterilants used to reprocess flexible endoscopes can injure mucous membranes if not thoroughly rinsed from the endoscope (Rutala et al., 2008).* *After high-level disinfection, the endoscope must be thoroughly rinsed and the channels flushed with sterile, filtered, or tap water to remove the disinfectant*/*sterilant (Petersen et al., 2011)."*

Chemicals used for high level disinfection tend to be pungent and severely irritating , require staff to wear full protective Equipment (PPE) including latex gloves and face masks to prevent serious health injury from the chemicals, some of them are corrosive to instruments , and some require an additional neutralisation step prior to disposal⁶.
⁶ See ref 1 pages 10-15 for advantages and disadvantages of commonly used Hdl's Some such as PAA are potentially explosive. In all cases, the instrument needs to be rinsed free of the disinfectant as part of the disinfection step.

The necessity for separate cleaning and sterilizing baths and for efficient rinsing between use of them arises since enzymes being proteins are denatured by all known disinfecting agents and since disinfecting agents are affected by enzymes (as enzymes are proteins).

Accordingly prior hereto it has proved impossible to provide a "single bath" for adequate cleaning and sterilizing treatment, although a two part system involving an enzyme treatment followed by addition of a phenolic disinfectant in the same bath has been proposed, but not widely adopted.

The disinfection/sterilising step typically adds up to a further 20 minutes to a reprocessing cycle.

WO 01/76647 A1, by the applicant of the present application, was based on our observation that procedures in use prior to 2001AD, while effective for preventing cross infection between patients, in fact exposed medical and/or hospital staff to then previously unrecognised health and safety risks. By virtue that the enzymes of the pre-soak bath digested the biological secretions holding the microorganisms, thus releasing them within the bath, and surfactants efficiently dispersed them, the fluid content of the pre-wash bath is itself readily contaminated to high levels with infectious material. Contrary to the belief of some hospital staff, the enzymes did not kill bacteria but rather release them. The present inventors had measured bacterial counts in excess of 10⁹ forming units ("cfu") per sq. cm. on instruments entering the first bath, and had concluded that Staff were therefore at risk of infection (i) from splashes from the first bath either during scrubbing to release contaminants or during draining the first bath (or from splashes if an instrument is accidentally dropped into the bath), (ii) from glove failures (latex gloves have a "pinhole" failure rate of about 12%), (iii) from accidental glove immersion above the wrist line, (iv) from finger stick incidents in the bath resulting in glove and sometimes dermal penetration, (v) from aerosols created by brushes and syringes. In addition the wall surface of the first bath remained contaminated after the bath has been emptied and if not itself disinfected may be handled by unprotected staff.

WO 01/76647 A1 disclosed a liquid composition intended for use as a pre-cleaning or cleaning bath. The composition was intended to reduce microbial inoculum on a medical instrument contaminated with an organic load including a protein, and comprised in brief a protease; a biocidal quat biocide; and an activity protector. Preferred embodiments included a non-ionic surfactant. It will be understood that quat biocides are instantaneously deactivated by protein and certain ions such as those found in hard water and therefore it was surprising that they could be employed in an environment of protein soiled instruments. Even more surprisingly, enzymes are also proteins and would be expected to denature the quat and to be inactivated themselves by a quat. That was avoided in our previous invention by combination with boron Activity Protectors. In the presence of Activity Protectors, the quat enabled the solution to pass the TGA Grade A test for "Hospital Grade disinfection" giving an 8 or 9 log reduction in inoculum density within 8 minutes. While compositions as described in that specification were effective in disinfecting the pre-soak or cleaning solutions so that they were no longer a health hazard for workers using them, that is to say killed bacteria released into the solution by the enzyme treatment, and disinfected the bath walls, the level of disinfection achieved (hospital Grade A) was not sufficient to disinfect the instruments being cleaned sufficiently for them to be able to be reused without undergoing a further separate disinfection or sterilization process. No Quats have previously provided High Level Disinfection. The instruments after treatment by compositions according to that invention were required to undergo subsequent High Level Disinfection before reuse.

Furthermore compositions according to our Patent to application WO 01/76647 A1 were not sufficiently effective as cleaners for medical instruments to pass the cleaning efficacy standards set in ISO 15883 and therefore gain regulatory/commercial acceptance for that purpose. In order to meet ISO 15833 instruments pre-cleaned in accordance with WO 01/76647 A1 had to be subsequently cleaned in an AER or cleaning bath able to meet that standard. Acceptable Cleaning efficacy requires cleaning of simulated soils from ISO 15883/5 complying indicators (such as "BROWNE"^{©} test strips (Steris Corp product), or similar, in the cleaning bath, and ensuring that the screen printed soil pattern printed on the test strip is removed from the substrate during the commercial cleaning cycle. Formulations according to WO 01/76647 A1 failed to comply with ISO 15583 and remove the soil from a test strip within a commercial 3-5 mins at 50°C at a concentration of 3-10ml/L.

Despite extensive R&D efforts, we were unable to improve the cleaning efficacy of the Enzyme/ Boron combination according to our previous invention without either the enzymes destroying the disinfection efficacy of the quat biocide in our compositions or the quat denaturing the enzymes sufficiently to be commercially acceptable. Prior to this invention, some 15 years later than the priority date of our previous invention, it has remained an unachievable goal to obtain satisfactory cleaning of medical instruments in a single bath while at the same time to achieve a sufficiently high level of disinfection as would permit the instruments to be safely reused. Our patent US 9,023,778 disclosed a cleaner which was not a disinfectant and which excluded surfactants (and thus excluded quats, all of which are surfactants).

### OBJECTS OF THE INVENTION

It is an object of the present invention to avoid or ameliorate the above discussed disadvantages of prior art, or at least to provide a commercial alternative to the prior art. In a preferred embodiment it is an object to provide improved means for High Level Disinfection which reduce one or more of the OH&S risks to staff, the corrosion risks to instruments and surroundings or the environmental disposal problems associated with currently used HLD disinfectants.

It is another object of preferred embodiments of the present invention to provide for satisfactory cleaning of a medical instrument in a single bath while concurrently achieving a sufficiently high level of disinfection as would permit the instruments to be safely reused and returned for use in a shorter time than is possible with prior art cleaning and disinfecting methods.

A further object of preferred embodiments is to obviate the need for rinsing between cleaning and disinfecting during instrument reprocessing whereby to save water, time and perhaps energy. Preferred embodiments of the invention also address the risk of cross infection of instruments by virtue of multiplication of microorganisms, if any, which remain on the bath walls after each cycle of instrument cleaning.

It is an object not according to the invention to provide simple means for cleaning surfaces. It is an object not according to the invention to provide simple means for achieving Disinfection of surfaces which require to be disinfected. It is an object of yet other embodiments of the invention to provide simple means for simultaneously cleaning surfaces while achieving High Level disinfection of those surfaces.

In some cases instruments may not be required to be sterilised. For example with spatulas, and holders which do not penetrate the body tissue, hair dressing implements and the like (which are not according to the invention), it may be sufficient to merely sanitise or disinfect the instruments to an appropriate standard. In such cases it would be desirable to provide a cleaning and disinfecting treatment capable of meeting the required standards with a single composition. In some embodiments not according to the invention desire ably the treatment could be applied to surfaces for example operating theatre surfaces or food preparation surfaces by spray or wipe to clean and disinfect those surfaces, with residues, if any, being subsequently removed by suitable means.

### BRIEF DESCRIPTION OF THE INVENTION

The methods according to claim 1 of the present invention use a liquid composition for achieving High Level Disinfection of a surface to which it is applied, wherein the surface is a surface of a medical instrument, said composition comprising an enzyme; a biocidal quaternary ammonium biocide, an anionic hydrotrope and a boron compound.

Those skilled in the art would hitherto have considered that enzymes would be incompatible with quats and that an anionic hydrotropes would be incompatible with both enzymes and quats. Moreover there would be no reason to suppose that such a combination would yield High Level Disinfection since neither enzymes, nor quats, nor hydrotropes alone are able to do so and textbooks teach that enzymes and especially anionic compounds would interfere with disinfection by quats. The composition used in the method of the invention also contains an enzyme activity protector or protection system which is a boron compound such as described in our earlier application WO 01/76647 A1 which did not include an anionic hydrotrope.

Compositions used in the methods of the invention achieve High Level Disinfection ("HLD") by means which are benign and relatively free of OH&S risks and environmental risks in comparison with those currently recommended for achieving this level of disinfection.

A liquid composition used in a method of the invention is effective for removing contamination by an organic load including a protein, if any, on said surface.

Preferred embodiments of the invention can remove soil from a test strip in less than 10 mins at 40°C without agitation at 3-10 ml/L dilutions. Nothing in the prior art suggests that such a combination might comply with ISO 15583 and remove the soil from a test strip within a commercial 3-5 mins at 50°C at a concentration of 3-10ml/L.

The method of the present invention uses a liquid composition intended for use in a bath for reducing inoculum on a surface of a medical instrument contaminated with an organic load including a protein while concurrently achieving High Level Disinfection of said instrument, said composition comprising an enzyme; a biocidal quaternary ammonium biocide, an anionic hydrotrope and a boron compound.

Hitherto cleaning and disinfecting has required a three step sequential process.

In some embodiments, the method of the invention uses a shelf stable liquid concentrate intended to be diluted by from 10:1 to 200:1 for use in a bath for reducing inoculum on a medical instrument contaminated with an organic load including a protein.

In some embodiments, the method of the invention uses a composition wherein the quat is present at a concentration which when diluted for use is below the Minimum Inhibitory Concentration ("MIC") of the quat to any challenge microorganism indicated in TG054

In some embodiments, the method of the invention uses a composition wherein the anionic hydrotrope is selected from alkali metal xylenesulphonates, and alkali metal cumene sulphonates, other alkali metal alkylarylsulphonates and combinations thereof

In some embodiments, the method of the invention uses a composition wherein the biocidal quat acts also acts as a cationic surfactant.

It will be very surprising to those skilled in the art that an enzyme and a quat can be combined within a shelf life stable composition in a manner which leaves the enzyme effective to digest the protein on the instrument while at the same time achieving High Level disinfection. (See e.g.US 6235692 column 6 lines 57-58 *"quaternary ammonium salts are not acceptable anti-microbial agents because they are not compatible with enzymes."* It is even more surprising that, contrary to text book teaching the cationic quat is not neutralised and deactivated by the anionic hydrotrope, both in the concentrate and upon dilution. It will be still more amazing to those skilled in the art that High Level disinfection can be achieved in a bath in which the dilution of the quat is such that its concentration is below that of its minimum inhibitory concentration ("MIC") against any organism of the quat as a disinfectant. Given that enzymes are not of themselves disinfectants, and none of the other components can produce High Level disinfection at any concentration, it follows that the High Level disinfection produced in a bath according to the present invention is not produced by the quat alone, but is a result of entirely unexpected synergistic interaction between the components.

Even more surprisingly, in preferred embodiments of the invention the quat biocide is in a composition in the form of a liquid concentrate (which can be diluted with water before use from 1:20 to 1:1000) which retains its biocidal activity in prolonged shelf-storage in contact with one or more enzymes which are also proteins which normally would be expected to quickly deactivate the quat biocide, and in combination with an anionic compound (the hydrotrope) which would also be expected to quickly deactivate the quat. Surprisingly, also, the enzymes are not irreversibly denatured. The liquid concentrate is readily diluted with water for use and provides a benign bath in comparison with prior art high level disinfectants in common use.

The method of the invention uses a composition including an enzyme activity protector which is a boron compound

In preferred embodiments of the invention, the cleaning and Disinfection are conducted in a single bath.

The invention consists in a method of cleaning a surface contaminated with an organic load including a protein while simultaneously achieving high level disinfection of the surface, wherein the surface is a surface of a medical instrument and wherein the method comprises the step of treating the surface with a liquid composition comprising: an enzyme, a biocidal quaternary ammonium biocide, an anionic hydrotrope and a boron compound. The surface may be that of a medical instrument or part thereof. In some embodiments of the invention, the method uses a composition where the ratio of anionic hydrotrope to quaternary compound is at least two parts of anionic hydrotrope to one part of quat.

More preferably, the ratio of anionic hydrotrope to quaternary compound is from five to ten parts of anionic hydrotrope to one part of quat.

It was also surprisingly and unexpected that when diluted from 1:20 to 1:100 the proteolytic activity of the preferred formulations with added quaternary biocide is substantially higher compared to the formulations without the quaternary biocide. The increase in the assayed activity is as high as 50%. The shelf life stability of enzymes in the formulations remain virtually unchanged (+/- 5-7%) over 24 months.

Unless the context clearly requires otherwise, throughout the description and the claims, the words 'comprise', 'comprising', and the like are to be construed in an inclusive sense as opposed to an exclusive or exhaustive sense; that is to say, in the sense of "including, but not limited to".

In Australia, environmental surface disinfectants (not to be used on medical instruments/devices) are graded according to tests specified by the TGA in order of decreasing efficacy as Grade B "Hospital Dirty", Grade A "Hospital Clean", Grade C "household/commercial". The TGA tests are specified as TGO 54. Similar tests and classifications are applicable in other countries. The term "hospital grade" disinfectant" is herein used to refer to disinfectants passing the Grade A test, i.e. a "hospital grade" disinfectant must be at least Hospital Grade A. The TGA specification requires that a "hospital grade disinfectant is able to give at least an 8 or 9 log reduction in inoculum density within 8 minutes".

"High Level disinfection" is defined by the Australian TGA.as a disinfectant that kills **all** microbial pathogens except large numbers of bacterial endospores when used as recommended by its manufacturer (TGA order No 54), that is to say gives at least 6 log reduction against Mycobacteria (which are very tough) and non-enveloped viruses.

In contrast to the compositions of our previous invention (WO 01/76647 A1) which could not achieve High Level disinfection of the instruments for which a subsequent separate disinfection or sterilizing treatment was required, the present invention achieves High Level disinfection in a bath which is also effective for use in cleaning a medical instrument contaminated with an organic load including a protein. Unlike presently approved methods for disinfecting instruments the chemicals employed in this invention are relatively benign and do not carry the Occupational Health risks associated with use of Glutaraldehyde, Orthophalaldehyde ("OPA"), Peracetic Acid ("PAA") , Hydrogen Peroxide or the like.

Moreover In contrast to the compositions of our previous invention which were unable to remove test soils from a test strip within 60 minutes at 50°C, methods according to the present invention can achieve that in less than 10 mins at 40°C and in 15-20 minutes at room temperature, and can reprocess instruments without requiring a further 20 minute high level disinfection step.

### DETAILED DESCRIPTION OF THE INVENTION

### Anionic Hydrotrope

An essential feature of the present invention is the inclusion of an anionic hydrotrope. A hydrotrope is a compound that solubilises hydrophobic compounds in aqueous solutions.

Desirably the hydrotrope is selected from the group consisting of water soluble anionic hydrotropes of formula:

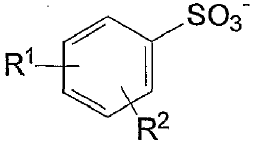

And more preferably of the formula: 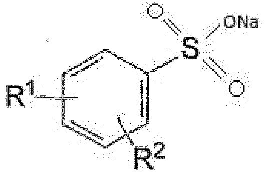 and having no alkyl side chains greater than six carbons in length. In preferred hydrotropes R¹ and R² are independently alkyl groups from 1 to six carbons, preferably from one to four carbons, and more preferably from one to two carbons, although R¹ or R² may optionally be hydrogen. Very highly preferred hydrotropes are water soluble xylene sulphonate (R¹ and R² are methyl) and cumene sulphonate (R¹ is isopropyl, R² is hydrogen) salts.

Examples of suitable anionic hydrophobic compounds include sodium xylenesulphonate ("SXS"), and sodium cumene sulphonate ("SCS"). However other suitable anionic hydrotropes include sodium-2-ethyl hexylsulphate, phosphate ester of oxyethylated phenol, amine alkylaryl sulphonate, linear alkyl naphthalene sulphonate, sodium dihexyl sulphosuccinate, and sodium dodecylbenzene sulphonate. Desirably the anionic hydrotrope is present in a concentration sufficient that the quaternary ammonium biocide is effective in use to provide "High Level" disinfection (as herein defined) of the bath in the presence of the at least one enzyme and of a typical proteinaceous load in the bath. The ratio of anionic hydrotrope to quat is at least 2:1, more preferably 5:1.

### Activity Protector

A boron "activity protector" is present, which is known to be effective in stabilizing enzymes in liquid aqueous solutions, including enzyme stabilizing compounds and systems, (2) selected "micelle inhibitors", and mixtures of (1) and (2). In preferred embodiments of the invention the "activity protector" is a suitable concentration of boron anions. Other reversible boron enzyme inhibitors could be suitable in this application for example phenyl boronic acid and similar compounds described in EP 0707642A1. Desirably these are solvated in a polyol and may be combined with enzyme stabilizing synergists or adjuvants forming an enzyme stabilizing system. "Micelle inhibitors" include species known to modify as well as to inhibit micelle formation and may be selected from water miscible solvents such as C1 C6 alkanols, C1 C6 diols, C2 C24 alkylene glycol ethers, alkylene glycol alkyl ethers, and mixtures thereof. A highly preferred micelle inhibitor is di-(propylene glycol) methyl ether ("DPM") and analogues thereof which modify micelle formation. It is especially preferred to combine the use of borate ions with DPM which has been found by the present inventor synergistically to enhance the biocidal activity protection conferred on the quat. biocide without irreversibly denaturing the enzyme.

### Quat

It is highly preferred that the quat biocide is an aryl quat compound, preferably benzalkonium halide. Other biocidal quaternary ammonium compounds could be used.

### Enzymes

It is well known that enzymes may become denatured in storage, in the presence of other enzymes, and/or in the presence of antagonistic anions such as for example anionic surfactants, quaternary ammonium compounds and detergency "builders". A number of enzyme stabilizing systems have been developed and are well known in the enzyme formulation art. An example of an "enzyme stabilizing system" is a boron compound (e.g. boric acid ) which in the past has been used alone or with selected other adjuvants and or synergists (e.g. polyfunctional amino compounds, antioxidants, etc.) to protect proteolytic and other enzymes in storage and in various products. It has been theorised that an enzyme stabilizing system such as boron and calcium form intramolecular bonds which effectively cross-link or staple an enzyme molecule so as to hold it in its active spatial configuration. Enzyme stabilizers have not hitherto been used to protect the biocidal activity of a quat. biocide. The present invention is based on the surprising discovery that at least some enzyme stabilizing systems are effective in protecting the biocidal activity of quat. biocides in the presence of protein.

The present invention includes a boron "activity protector" of the kind discussed in our patent specification WO 01/76647 A1, e.g. boron in a ratio to quat. biocide chosen to substantially to minimise the Minimum Inhibitory Concentration ("MIC") of quat. biocide in the presence of the enzymes in the formulation and at a given level of protein load. MIC is a measure of the minimum concentration of the biocide which succeeds in preventing bacterial growth in a culture during a specified time period, for example 24 hrs. Details of the MIC test are shown in Bailey & Scott "Diagnostic Microbiology", 8.sup.th edition, 1990 at page 177. The TGA tests are specified at TGO 54 annexed. MIC tests referred to herein are conducted over 24 hrs.

In the present case in which an enzyme is present in addition to the quat. biocide and in which it is desired to retain the enzymatic activity of the enzyme as well as the biocidal activity of the quat, biocide then the quantity of "activity protector" required will need to be greater than that required merely to protect the enzyme and will need to be sufficient both to stabilise the enzyme and protect the biocidal activity of the quat. biocide. Moreover as the composition is anticipated to come into contact with an external proteinaceous load (from contaminants in the surgical instruments bath) then the "activity protector" concentration will need to be greater still.

The inventor discovered that boron surprisingly protects a quaternary biocide from deactivation by a protein in such a way and to such an extent that the MIC of the biocide is not increased in the presence of a protein. In preferred embodiments of the invention the MIC is dramatically reduced, for example, more than halved notwithstanding the presence of up to 2 wt. % based on the weight of solution, of protein. This allows the formulation of a wide range of new and useful compositions which remain effective as disinfectants or antibacterials in circumstances in which the prior art would be significantly less effective or not effective at all.

The invention also enables storage-stable liquid biocidally effective compositions to be prepared with a lower concentration of quat. biocide and at much lower cost. By "shelf stable" is meant that the composition retains at least 50% of its biocidal efficacy after 12 months storage in a sealed container at 18 - 25°C. Preferred embodiments of the invention retain better than 98% biocidal efficacy under these conditions.

Without wishing to be bound by theory, the inventor speculates that polymeric borate ions associate with the cationic quat. biocide, thus protecting the quat biocide from combining with proteins. When the formulation is diluted the polymeric ions become unstable and release the quat biocide for disinfection. Alternatively, it may be that the biocidal activity of the quat. biocide significantly relates to denaturing proteins of cell membranes and that boron complexes with charged groups of non-living proteins and prevents wasting quat. on denaturing non-living proteins. However as enzymes are structurally quite different from quat. biocides, and as the complete mechanism by which quat. biocides kill bacteria is also uncertain, it was not previously predictable that any enzyme stabilizer would be effective in maintaining the biocidal activity of a quat. biocide (an enzyme antagonist). The mechanism by which the activity of the quat biocide is maintained may be different from that whereby the enzyme is stabilised.

### EXAMPLES OF THE INVENTION

Several formulations with varying concentration of hydrotropes, various groups of commercially available proteases and quaternary amine (biocidal active) suitable for use in the method of invention were prepared as shown in Table 1 annexed hereto. Some of the formulations are with or without non-ionic surfactants

The formulations of table 1 are identified by designations126-8 to 126-20 and all are examples of multi-enzyme cleaning and sanitising products for use in methods according to the invention in manual baths and AER medical instrument reprocessors. The preferred use concentration is between 5 mL/L and 20 mL/L and at temperatures from 25°C to 60°C (maximum temperature to which flexible endoscopes could be exposed).

The cleaning efficacy and foaming properties of the composition was then tested and compared with formulations identified as 84-0, 84-2, 84-4 and 84-10 made in accordance with WO 01/76647 A1 and shown in Table 2 annexed hereto

Tables 3, 4 and 5 annexed hereto demonstrate the cleaning efficacy of the formulations of Table 1. PF-126 formulations were diluted with distilled water at 25°C (table 3) , hot water at 50°C (table 4) or at 40°C (table 5) (to concentrations 2 mL/L, 5mL/L and 20 mL/L) in glass beakers. The temperature of the solutions were maintained in a water bath for the duration of testing. A test soil was introduced in the form of a Browne load check strip at the same time as a stopwatch was started. The test strips were monitored over time to identify how long it took the test soil to be completely removed from the Browne load check strip.

All samples in Table 2 (in accord with WO 01/76647 A1) failed to remove the test soil even at 50°C and a concentration of 20 mL/L within 60 minutes.

Formulations for use in methods according to the present invention on the other hand as shown in Tables 4 & 5, removed the test soil within commercially acceptable times even at static conditions.

The static cleaning used in the above experiments is the worst case scenario. With agitation/mixing of the solutions simulating the agitations encountered in AERS and washer disinfectors the cleaning efficacy speeds up markedly: for example formulation 126-8 cleans Browne Load Check at [25°C 5ml/l] in 31 minutes at static conditions, in 7 minutes at AER agitations and less than 4 minutes in an orbital shaker mimicking the agitations of washer disinfectors. The formulation 84-0 - the best performing out of the prior art formulations - could not clean Browne strip at 20ml/l 50°C in 60 minutes even in an orbital shaker.

Table 7 shows the proteolytic activity of the preferred formulations compared to formulations without a quaternary biocide. It can be seen rather surprisingly and unexpectedly that the proteolytic activity of the formulations containing a quaternary biocide is substantially higher.

Table 8 shows the biocidal activity of preferred formulations against S.aureus ATCC 6538 and P.aeruginosa ATCC 15442. It can be seen that the biocidal activity is retained even at a high dilution factor of 1:1000.

Table 9 shows stability data for some preferred formulations. Each formulation is tested for proteolytic activity when first made and is then stored at 25°C and 45°C. After 220 days in storage, the proteolytic activities of the formulations were retested. Storage at 45°C for 220 days is equivalent to storage for 700 days (about 2 years) at 25°C. It is generally recognised in the art that loss of up to 50 % of proteolytic activity on storage is acceptable.

Examples of some formulations that combine proteases and quats, proteases and hydrotropes, and quats and hydrotropes are shown in Table 10. Formulation 126-8 is for use in accordance with the methods of the invention. The other formulations are either not stable (hazy) or exhibit unacceptably poor bactericidal efficacy (greater than 40 minutes as per the test protocol in the table) or unacceptably poor cleaning (greater than 30 minutes as per the test protocol in the table).

### BIOCIDAL EFFICACY

Compositions 126-8,126-9,126-13,126-14 were evaluated for biocidal efficacy as per EN 1276 (biocidal) and EN 14348 (turbeculocidal). Table 6 annexed summarises the treatment envelop (conc, temp, time) required to achieve HLD for these formulations. As expected, Mycobacteria (TB) presented the greatest challenge. The increased concentration of QUATs improved the bactericidal efficacy. At the same time, the effect of water hardness was not as detrimental to quat activity as a person skilled in the art would expect indicating that enzymes might be symbiotic with quat in achieving high levels of kill.

A sample of the product diluted with hard water is added to a test suspension of mycobacteria in a solution of an interfering substance. The mixture is maintained at one of the temperatures and the contact times specified. At the end of this contact time, an aliquot is taken; the bactericidal and/or the bacteriostatic action in this portion is immediately neutralized or suppressed by a validated method.

### TEST CONDITIONS

Test organism(s) *Mycobacterium terrae (ATCC 15755)*
Test temperature(s) 40°C, 45°C, 50°C
Contact time(s) 5 min - 30 min
Product diluent(s) 0 ppm and 300 ppm hard water
Interfering substance(s) Clean conditions = 0.3 g/L bovine serum albumin
Dirty conditions = 3 g/L bovine serum albumin + 3mL/L erythrocytes

### CONTROLS AND VALIDATIONS

All controls and validations were within the basic limits (EN 14348).

### RESULTS

### See Table 6 annexed.

When used at 40°C, P126-4 is bactericidal within 30 minutes.

When used at 45°C, P126-4 is bactericidal within 15 minutes.

When used at 50°C, P126-4 is bactericidal within 5 minutes.

Using soft water (preferably RO water or distilled water) for dilution is recommended. It is of note that most potable water supplies have water hardness of below 50 ppm.

Since mycobacteria are regarded as one of the greatest challenges in the hierarchy of pathogens (inferior only to only endobacterial spores) the above results indicate that the formulation is capable to disinfecting instruments to High Level Disinfection as per TGO54. Similar results have been obtained with formulas PF-126-5,-6, -7,- 8, -9 & -13, -14.

As will be apparent to those skilled in the art from the teaching hereof compositions for use in methods according to the invention may be modified to provide lower levels of disinfection such as "Intermediate Level Disinfection", "Hospital Grade Disinfection", Safe to Handle" Disinfection" or as a

Sanitiser if the intended use and applicable standards permit, but these methods are not according to the invention. Compositions are also useful for cleaning and/or disinfection of other surfaces in hospitals, medical and dental practices, nursing homes or the like - for example chamber pots, trays, instrument transport trolleys and other large equipment - and for cleaning and/or disinfection food preparation areas, food utensils, dispensing equipment, cool rooms and the like, or fabrics and the like such as are treated in hospital laundries. These methods are not according to the invention.

**Table 1: Example Formulations**

| **Ingredient/ Composition ID** | **126 -8** | **126 -9** | **126 -10** | **126- 11** | **126- 12** | **126- 13** | **126- 14** | **126- 15** | **126- 16** | **126- 17** | **126- 18** | **126- 19** | **126-20** |
|---|---|---|---|---|---|---|---|---|---|---|---|---|---|
| DPM⁷ | 4 | 1 | 8 | 4 | 4 | 4 | 4 | 4 | 4 | 4 | 4 | 4 | 4 |
| Sodium Cumene Sulphonate⁸ | 10 | 10 | 10 | | | | | 10 | 10 | 10 | 10 | 10 | 10 |
| Sodium xylene sulphonate | | | | 12 | | | | | | | | | |
| Potassium xylene sulphonate | | | | | 8 | | | | | | | | |
| Sodium toluene sulphonate | | | | | | 8 | | | | | | | |
| Sodium salt dodecylbenzene sulphonic acid | | | | | | | 8 | | | | | | |
| Boron as boric acid | 2 | 2 | 1 | 1 | 1 | 2 | 2 | 1 | 0.8 | 2 | 2 | 2 | 2 |
| Serine Protease (Savinase 16L⁹) | 3 | 3 | | 3 | 3 | 2 | 2 | | | 2 | 3 | 3 | 3 |
| Cysteine Protease Papain | | | 2 | | | | | 2 | | | | | |
| Metalloprotease Endopeptidase Trypsin | | | | | | | | | 2 | | | | |
| Amylase Termamyl 300L | 1 | 1 | | | 1 | 1 | 1 | | 1 | 1 | 1 | 1 | 1 |
| Teric GN9 | 1 | 1 | 1 | | | | | 1 | 1 | 0 | 1 | 1 | 1 |
| Guardiquat 1450 (as 100% active)¹⁰ | 2 | 4 | | | | 2 | 4 | | | 2 | 2 | 2 | |
| Barquat MB-80 (benzalkonium chloride) | | | 2 | 2 | 2 | | | | | | | | 2 |
| Carboquat^{™} MW-50 Didecyl Dimethyl Ammonium Carbonate | | | | | | | | 3 | 2 | | | | |
| Cold potable water | qc | qc | | | | | | | | | | qc | qc |
| pH (1:100 dilution) | 8.2 | 9 | 7.8 | 8.6 | 9.3 | 6.9 | 9.2 | 9.3 | 8.1 | 7.7 | 8.4 | 8.2 | 9.0 |

| | | | | | | | | | | | | | |
|---|---|---|---|---|---|---|---|---|---|---|---|---|---|
| ⁷ Diproplene Glycol methyl ether e.g. "Dowanol DPM" ex Dow Chemicals ⁸ Sodium Cumene Sulphonate e.g. ex Stepan ⁹ Savinase, Lipolase , Puradex and Termamyl ex Novazyme ¹⁰ Quats ex Albright and Wilson. | | | | | | | | | | | | | |

**Table 2: Formulation number and corresponding composition for comparative formulations based on compositions in accord with WO 01/76647 A1**

| **Ingredient** | **84-0 (w/v)%** | **84-2 (w/v)%** | **84-4 (w/v)%** | **84-6 (w/v)%** | **84-10 (w/v)%** |
|---|---|---|---|---|---|
| DPM | 4 | 4 | 4 | 4 | 4 |
| Propylene glycol | 15 | 15 | 15 | 15 | 15 |
| Teric 168 | 6 | 6 | 6 | 6 | 6 |
| 4Na-EDTA | 1 | 1 | 1 | 1 | 1 |
| Sulfamic acid | 3 | 3 | 3 | 3 | 3 |
| Genamin LAP 100D | 10 | 10 | 10 | 10 | 10 |
| Barquat MB-80 | 1 | 3 | 5 | 7 | 11 |
| Savinase Ultra 16XL-NF | 10 | 10 | 10 | 10 | 10 |
| Distilled water | 50 | 48 | 46 | 44 | 40 |

**Table 3: Cleaning times (in minutes) for full digestion of Browne load check strip soil at 25°C.**

| **Concentration** | **PF-126-8** | **PF-126-9** | **PF-126-13** | **PF-126-14** |
|---|---|---|---|---|
| 2 mL/L | >60 | >60 | >60 | >60 |
| 5 mL/L | 31 | 27 | 34 | 30 |
| 20 mL/L | 17 | 16 | 12 | 19 |

**Table 4: Cleaning times (in minutes) for full digestion of Browne load check strip soil at 50°C.**

| **Concentration** | **PF-126-8** | **PF-126-9** | **PF-126-13** | **PF-126-14** |
|---|---|---|---|---|
| 2 mL/L | 35 | 36 | 43 | 46 |
| 5 mL/L | 16 | 19 | 26 | 24 |
| 20 mL/L | 9 | 9 | 9 | 9 |

**Table 5: Cleaning times (in minutes) for full digestion of Browne load check strip soil at 40°C.**

| **Concentration** | **PF-126-8** | **PF-126-9** | **PF-126-13** | **PF-126-14** |
|---|---|---|---|---|
| 2 mL/L | 43 | 43 | 50 | 52 |
| 5 mL/L | 21 | 21 | 21 | 21 |
| 20 mL/L | 9.5 | 9.5 | 9.5 | 9.5 |

**Table 6: Biocidal Efficacy Results**

| | | | **126-8** | **126-9** | **126-13** | **126-14** |
|---|---|---|---|---|---|---|
| **Dilution** | **Temp** | **Diluent** | **Pass at (min)** | | | |
| 10 mL/L | 40°C | 0 ppm | 30 | 20 | >30 | 20 |
| 10 mL/L | 40°C | 300 ppm | 30 | 20 | 30 | 30 |
| 20 mL/L | 40°C | 0 ppm | 20 | 15 | 20 | 15 |
| 20 mL/L | 40°C | 300 ppm | 20 | 15 | 20 | 15 |
| 10 mL/L | 45°C | 0 ppm | 15 | 15 | 15 | 10 |
| 10 mL/L | 45°C | 300 ppm | 15 | 10 | 15 | 10 |
| 20 mL/L | 45°C | 0 ppm | 10 | 10 | 10 | 10 |
| 20 mL/L | 45°C | 300 ppm | 10 | 5 | 10 | 5 |
| 10 mL/L | 50°C | 0 ppm | 5 | 5 | 5 | 5 |
| 10 mL/L | 50°C | 300 ppm | 5 | 5 | 5 | 5 |
| 20 mL/L | 50°C | 0 ppm | 5 | 5 | 5 | 5 |
| 20 mL/L | 50°C | 300 ppm | 5 | 5 | 5 | 5 |

**Table 7: Proteolytic activity**

| **Formulation** | **Proteolytic Activity (Au/ml)** | **Increase in Assayed Proteolytic Activity** |
|---|---|---|
| PF-126-8 (with quat) | 0.476 | 67% |
| PF-126-8 (without quat) | 0.317 | |
| PF-126-9 (with quat) | 0.484 | 70% |
| PF-126-9 (without quat) | 0.341 | |
| PF-126-13 (with quat) | 0.468 | 65% |
| PF-126-13 (without quat) | 0.303 | |

**Table 8: Biocidal activity of preferred formulations against S.aureus ATCC 6538 and P.aeruginosa ATCC 15442**

| | | | **126-8** | **126-9** | **126-13** | | **126-14** |
|---|---|---|---|---|---|---|---|
| **Dilution** | **Temp** | **Diluent** | **Pass at (min)** | | | | |
| S.aureus ATCC 6538 | | | | | | | |
| 1 mL/L (1:1000) | 40°C | 0 ppm | 20 | 25 | 20 | | 20 |
| 1 mL/L (1:1000) | 40°C | 300 ppm | 35 | 40 | 40 | | 40 |
| 5 mL/L (1 :200) | 40°C | 300 ppm | 20 | 20 | 20 | | 20 |
| 5mL/L (1 :200) | 40°C | 0 ppm | 15 | 15 | 15 | | 15 |
| | | | | | | | |

| | | | **126-8** | **126-9** | | **126-13** | **126-14** |
|---|---|---|---|---|---|---|---|
| **Dilution** | **Temp** | **Diluent** | **Pass at (min)** | | | | |
| P.aeruginosa ATCC15442 | | | | | | | |
| 1 mL/L (1:1000) | 40°C | 0 ppm | 30 | 30 | 30 | | 30 |
| 1 mL/L (1:1000) | 40°C | 300 ppm | 45 | 45 | 45 | | 45 |
| 5 mL/L (1 :200) | 40°C | 300 ppm | 25 | 30 | 25 | | 25 |
| 5mL/L (1 :200) | 40°C | 0 ppm | 20 | 20 | 20 | | 20 |

**Table 9: Shelf life stability of preferred formulations:**

| | | **Proteolytic Activity (Au/ml)** | | |
|---|---|---|---|---|
| **Formulations** | **Storage Temperature** | **0 Days** | **220 Days*** | **Loss on storage** |
| PF-126-8 | 25°C | 0.320 | 0.31 | 3.1% |
| | 45°C | 0.36 | 0.32 | 9.9% |
| PF-126-9 | 25°C | 0.34 | 0.32 | 5.3% |
| | 45°C | 0.36 | 0.28 | 21.3% |
| PF-126-13 | 25°C | 0.32 | 0.32 | 1.2% |
| | 45°C | 0.33 | 0.29 | 12.4% |
| PF-126-14 | 25°C | 0.33 | 0.32 | 0.6% |
| | 45°C | 0.33 | 0.28 | 16.2% |

| | | | | |
|---|---|---|---|---|
| * 220 Days at 45°C is equivalent to storage for about 2 years at 25°C. | | | | |

**Table 10: Examples of prior art formulations:**

| | Formulation of the invention | Prior art formulations | | | | | | | |
|---|---|---|---|---|---|---|---|---|---|
| Ingredient/ Composition ID | 126-8 | 126-81 | 126-82 | 126-83 | 126-84 | 126-85 | 126-86 | 126-87 | 126-88 |
| Includes: P=Protease; Q=quat; H=hydrotrope | P+Q+H | P+Q; No H | P; No Q; No H | Q+H; No P | Q; No H; No P | H+Q; No P | H; no Q ; No P | P+H; NoQ | P; No H; No Q |
| DPM^{[7]} | 4 | 4 | 4 | 4 | 4 | 4 | 4 | 4 | 4 |
| Sodium Cumene Sulphonate^{[8]} | 10 | 0 | 0 | 10 | 0 | 10 | 10 | 10 | 0 |
| Boron as boric acid | 2 | 2 | 2 | 2 | 2 | 2 | 2 | 2 | 2 |
| Serine Protease (Savinase 16L^{[9]}) | 3 | 3 | 3 | 0 | 0 | 0 | 0 | 3 | 3 |
| Cysteine Protease Papain | | | | | | | | | |
| Metalloprotease Endopeptidase Trypsin | | | | | | | | | |
| Amylase Termamyl 300L | 1 | 1 | 1 | 1 | 1 | 1 | 1 | 1 | 1 |
| Teric GN9 | 1 | 1 | 1 | 1 | 1 | 1 | 1 | 1 | 1 |
| Guardiquat 1450 (as 100% active)^{[10]} | 2 | 2 | 0 | 2 | 2 | 2 | 0 | 0 | 0 |
| Cold potable water | qc | qc | qc | qc | qc | qc | qc | qc | qc |
| Cleaning efficacy expressed as time (in minutes) to clean Browne STF Load Check at 40°C, static conditions | 15 | >120 | 25 | >120 | >120 | 50 | 80 | 15 | 50 |
| Pass/fail cleaning test | P | F | P | F | F | F | F | P | F |
| Bactericidal properties expressed as time (min) required to pass suspension test as per EN1278 against P.aeruginosa ATCC15442. 1:100 dilution, 40°C | 10 | 20 | >180 | 20 | 20 | 40 | >180 | >180 | >180 |
| Pass as HLD? | P | P | F | P | P | P | F | F | F |
| Appearance | clear liquid | hazy liquid | hazy liquid | clear liquid | clear liquid | clear liquid | clear liquid | clear liquid | clear liquid |
| Loss of protease activity on 30 days storage at 45°C (%) | 6 | 14 | 15 | na | na | na | na | 9 | 20 |

| | | | | | | | | | |
|---|---|---|---|---|---|---|---|---|---|
| oplene Glycol methyl ether e.g. "Dowanol DPM" ex Dow Chemicals ⁸Sodium Cumene Sulphonate e.g. ex Stepan ⁹Savinase, Lipolase , Puradex and Termamyl ex Novazyme ¹⁰Quats ex Albright and Wilson. | | | | | | | | | |

## Claims

1. A method of cleaning a surface contaminated with an organic load including a protein while simultaneously achieving high level disinfection of the surface, wherein the surface is a surface of a medical instrument and wherein the method comprises the step of treating the surface with a liquid composition comprising: an enzyme, a biocidal quaternary ammonium biocide, an anionic hydrotrope and a boron compound.

2. A method according to claim 1, which method achieves at least 6 log reduction against Mycobacteria and non-enveloped viruses.

3. A method according to claim 1 or claim 2, wherein the enzyme is a protease.

4. A method according to any one of claims 1 to 3, wherein the quaternary ammonium biocide is an aryl quat compound.

5. A method according to claim 4, wherein the aryl quat compound is a benzalkonium halide.

6. A method according to any one of claims 1 to 5, wherein the anionic hydrotrope is selected from the group consisting of alkali metal xylenesulphonates, alkali metal cumene sulphonates, other alkali metal alkylarylsulphonates and combinations thereof.

7. A method according to any one of claims 1 to 5, wherein the anionic hydrotrope is selected from the group consisting of sodium xylenesulphonate and sodium cumene sulphonate.

8. A method according to any one of claims 1 to 5, wherein the anionic hydrotrope is of the formula: 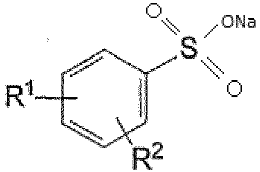 wherein R¹ and R² are independently alkyl groups from 1 to 6 carbons or hydrogen.

9. A method according to any one of the preceding claims wherein the boron compound is boric acid.

10. A method according to any one of claims 1, 2 and 4 to 9, wherein the composition comprises a C1 to C6 diol.

11. A method according to claim 10 wherein composition comprises borate ions and di(propylene glycol) methyl ether.

12. A method according to any one of the preceding claims, wherein cleaning and disinfection are conducted in a single bath.

## Patentansprüche

1. Verfahren zur Reinigung einer Oberfläche, die mit einer organischen Belastung kontaminiert ist, die ein Protein einschließt, während gleichzeitig eine High-Level-Desinfektion der Oberfläche erreicht wird, wobei die Oberfläche eine Oberfläche eines medizinischen Instruments ist und wobei das Verfahren den Schritt der Behandlung der Oberfläche mit einer flüssigen Zusammensetzung umfasst, die Folgendes umfasst: ein Enzym, ein biozides quartäres Ammoniumbiozid, ein anionisches Hydrotrop und eine Borverbindung.

2. Verfahren nach Anspruch 1, welches Verfahren mindestens eine 6-log-Reduzierung gegen Mykobakterien und unbehüllte Viren erreicht.

3. Verfahren nach Anspruch 1 oder Anspruch 2, wobei das Enzym eine Protease ist.

4. Verfahren nach einem der Ansprüche 1 bis 3, wobei das quartäre Ammoniumbiozid eine quartäre Arylverbindung ist.

5. Verfahren nach Anspruch 4, wobei die quartäre Arylverbindung ein Benzalkoniumhalogenid ist.

6. Verfahren nach einem der Ansprüche 1 bis 5, wobei das anionische Hydrotrop aus der Gruppe ausgewählt ist, die aus Folgenden besteht: Alkalimetallxylolsulfonaten, Alkalimetallcumolsulfonaten, anderen Alkalimetallalkylarylsulfonaten und Kombinationen davon.

7. Verfahren nach einem der Ansprüche 1 bis 5, wobei das anionische Hydrotrop aus der Gruppe ausgewählt ist, die aus Natriumxylolsulfonat und Natriumcumolsulfonat besteht.

8. Verfahren nach einem der Ansprüche 1 bis 5, wobei das anionische Hydrotrop die folgende Formel aufweist: 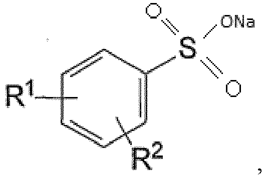 wobei R¹ und R² unabhängig Alkylgruppen von 1 bis 6 Kohlenstoffen oder Wasserstoff sind.

9. Verfahren nach einem der vorstehenden Ansprüche, wobei die Borverbindung Borsäure ist.

10. Verfahren nach einem der Ansprüche 1, 2 und 4 bis 9, wobei die Zusammensetzung ein C1- bis C6-Diol umfasst.

11. Verfahren nach Anspruch 10, wobei die Zusammensetzung Borationen und Di(propylenglycol)methylether umfasst.

12. Verfahren nach einem der vorstehenden Ansprüche, wobei die Reinigung und Desinfektion in einem einzigen Bad durchgeführt werden.

## Revendications

1. Méthode de nettoyage d'une surface contaminée par une charge organique comportant une protéine tout en réalisant simultanément un haut niveau de désinfection de la surface, dans laquelle la surface est une surface d'un instrument médical et la méthode comprenant l'étape de traitement de la surface par une composition liquide comprenant : une enzyme, un biocide à base d'ammonium quaternaire biocide, un hydrotrope anionique et un composé de bore.

2. Méthode selon la revendication 1, laquelle méthode permettant d'obtenir une réduction d'au moins 6 log des mycobactéries et des virus non enveloppés.

3. Méthode selon la revendication 1 ou la revendication 2, dans laquelle l'enzyme est une protéase.

4. Méthode selon l'une quelconque des revendications 1 à 3, dans laquelle le biocide à base d'ammonium quaternaire est un composé d'aryle quat.

5. Méthode selon la revendication 4, dans laquelle le composé d'aryle quat est un halogénure de benzalkonium.

6. Méthode selon l'une quelconque des revendications 1 à 5, dans laquelle l'hydrotrope anionique est choisi dans le groupe constitué par les xylène sulfonates de métaux alcalins, les cumène sulfonates de métaux alcalins, d'autres alkylarylsulfonates de métaux alcalins et des combinaisons de ceux-ci.

7. Méthode selon l'une quelconque des revendications 1 à 5, dans laquelle l'hydrotrope anionique est choisi dans le groupe constitué par le xylène sulfonate de sodium et le cumène sulfonate de sodium.

8. Méthode selon l'une quelconque des revendications 1 à 5, dans laquelle l'hydrotrope anionique répond à la formule : 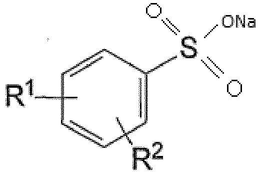 dans laquelle R¹ et R² sont indépendamment des groupements alkyle ayant de 1 à 6 carbones ou hydrogène.

9. Méthode selon l'une quelconque des revendications précédentes, dans laquelle le composé de bore est l'acide borique.

10. Méthode selon l'une quelconque des revendications 1, 2 et 4 à 9, dans laquelle la composition comprend un diol en C₁ à C₆.

11. Méthode selon la revendication 10, dans laquelle la composition comprend des ions borate et de l'éther méthylique de dipropylène glycol.

12. Méthode selon l'une quelconque des revendications précédentes, dans laquelle le nettoyage et la désinfection sont effectués dans un seul bain.
